# EUROPEAN PATENT SPECIFICATION

(11) **EP 1 310 508 B1**
(45) Date of publication and mention of the grant of the patent: **15.10.2008**
(21) Application number: 02080557.8
(22) Date of filing: 04.12.1997
(51) Int. Cl.: C07J 41/00, A61K 31/565, A61K 31/566, A61P 5/32

(54) **Estrogen sulfamate sulphatase inhibitors**
Östrogen-Sulfamate als Sulfataseinhibitoren
Sulfamates d'estrogène comme inhibiteurs de sulphatase

(30) Priority: 05.12.1996 GB 9625334
(43) Date of publication of application: 14.05.2003
(62) Divisional of application: 97947778.3
(73) Proprietor: Sterix Limited, Slough, Berkshire SL1 3XE (GB)
(72) Inventor: Reed, Michael J., c/o Sterix Limited, Slough, Berkshire SL1 3XE (GB); Potter, Barry V. L., c/o Sterix Limited, Slough, Berkshire Sl1 3XE (GB)
(74) Representative: Alcock, David

(56) References cited:
- WO-A-93/05064
- DE-A- 4 429 398
- US-A- 4 668 668
- L. WOO ET AL: "Active Site Directed Inhibition of Estrone Sulfatase by Nonsteroidal Coumarin Sulfamates" JOURNAL OF MEDICINAL CHEMISTRY., vol. 39, no. 7, 29 March 1996 (1996-03-29), pages 1349-1351, XP000652210 WASHINGTON US
- J. FISHMAN ET AL: "Studies on the Directive O-Methylation of Catechol Estrogens" JOURNAL OF THE AMERICAN CHEMICAL SOCIETY., vol. 89, no. 26, 1967, pages 7147-7148, XP002059346 DC US
- R. PETERS ET AL: "Analogues of [(Triethylsily)ethynyl]estradiol as Potential Antifertility Agents" JOURNAL OF MEDICINAL CHEMISTRY., vol. 31, no. 3, 1988, pages 572-576, XP002059347 WASHINGTON US
- R. RAJAN ET AL: "Estrogen Effects on NADH Oxidase and Superoxide Dismutase in Prepubertal Female Rats" STEROIDS., vol. 40, no. 6, 1982, pages 651-660, XP002059348 SAN FRANCISCO US
- M. CUSHMAN ET AL: "Synthesis, Antitubulin and Antimitotic Activity, and Cytotoxicity of Analogs of 2-Methoxyestradiol, an Endogenous Mammalian Metabolite of Estradiol That Inhibits Tubulin Polymerization by Binding to the Colchicine Binding Site" JOURNAL OF MEDICINAL CHEMISTRY., vol. 38, no. 12, 9 June 1995 (1995-06-09), pages 2041-2049, XP002055798 WASHINGTON US
- C. LOVELY ET AL: "2-(Hydroxyalkyl)estradiols: Synthesis and Biological Evaluation" JOURNAL OF MEDICINAL CHEMISTRY., vol. 39, no. 9, 26 April 1996 (1996-04-26), pages 1917-1923, XP002059350 WASHINGTON US
- CHEMICAL ABSTRACTS, vol. 107, no. 21, 23 November 1987 (1987-11-23), Columbus, Ohio, US; abstract no.: 190359, S. BROOKS ET AL: "A-Ring substituted estrogens as inhibitors of the MXT transplantable mammary ductal carcinoma" page 18 column 2 XP002059352 & CANCER RESEARCH, vol. 47, no. 17, 1987, pages 4623-4629,
- TOWNSLEY J D: "Further studies on the regulation of human placental steroid 3-sulfatase activity" ENDOCRINOLOGY, vol. 93, no. 1, 1973, pages 172-181, XP002068155
- CHEMICAL ABSTRACTS, vol. 068, no. 1, 1 January 1968 (1968-01-01), Columbus, Ohio, US; abstract no.: 009640, ADAMS J B: "Enzymic synthesis of steroid sulfates. V. Binding of estrogens to estrogen sulfotransferase" page 913 column 2 XP002068156 & BIOCHIMICA ET BIOPHYSICA ACTA, vol. 146, no. 2, 1967, pages 522-528,
- S. SCHWARZ ET AL: "Synthesis of estrogen sulfamates: Compounds with a novel endocrinological profile" STEROIDS., vol. 61, no. 12, December 1996 (1996-12), pages 710-717, XP002059351 SAN FRANCISCO US

## Description

The present invention relates to a compound.

In particular the present invention relates to a compound and to a pharmaceutical composition comprising the compound.

Evidence suggests that oestrogens are the major mitogens involved in promoting the growth of tumours in endocrine-dependent tissues, such as the breast and endometrium. Although plasma oestrogen concentrations are similar in women with or without breast cancer, breast tumour oestrone and oestradiol levels are significantly higher than in normal breast tissue or blood. *In situ* synthesis of oestrogen is thought to make an important contribution to the high levels of oestrogens in tumours and therefore specific inhibitors of oestrogen biosynthesis are of potential value for the treatment of endocrine-dependent tumours.

Over the past two decades, there has been considerable interest in the development of inhibitors of the aromatase pathway which converts the androgen precursor androstenedione to oestrone. However, there is now evidence that the oestrone sulphatase (E1-STS) pathway, i.e. the hydrolysis of oestrone sulphate to oestrone (E1S to E1), as opposed to the aromatase pathway, is the major source of oestrogen in breast tumours^{1,2}. This theory is supported by a modest reduction of plasma oestrogen concentration in postmenopausal women with breast cancer treated by aromatase inhibitors, such as aminoglutethimide and 4-hydroxyandrostenedione^{3,4} and also by the fact that plasma E1S concentration in these aromatase inhibitor-treated patients remains relatively high. The long half-life of E1S in blood (10-12 h) compared with the unconjugated oestrogens (20 min)⁵ and high levels of steroid sulphatase activity in liver and, normal and malignant breast tissues, also lend support to this theory⁶.

PCT/GB92/01587 teaches novel steroid sulphatase inhibitors and pharmaceutical compositions containing them for use in the treatment of oestrone dependent tumours, especially breast cancer. These steroid sulphatase inhibitors are sulphamate esters, such as N,N-dimethyl oestrone-3-sulphamate and, preferably, oestrone-3-sulphamate (otherwise known as "EMATE").

Some of the compounds disclosed in PCT/GB92/01587 are shown in Figure 1.

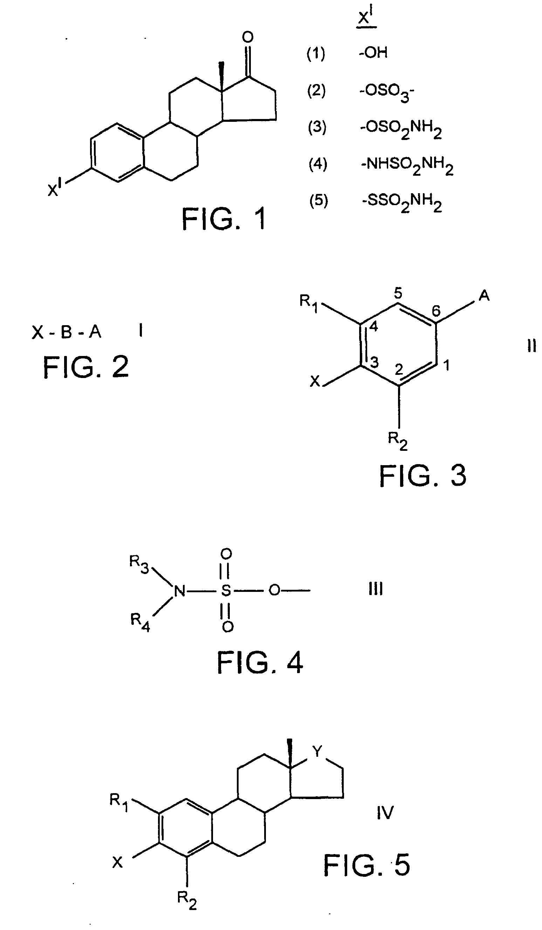

It is known that EMATE is a potent E1-STS inhibitor as it displays more than 99% inhibition of E1-STS activity in intact MCF-7 cells at 0.1 mM. EMATE also inhibits the E1-STS enzyme in a time- and concentration-dependent manner, indicating that it acts as an active site-directed inactivator^{7,8}. Although EMATE was originally designed for the inhibition of E1-STS, it also inhibits dehydroepiandrosterone sulphatase (DHA-STS), which is an enzyme that is believed to have a pivotal role in regulating the biosynthesis of the oestrogenic steroid androstenediol^{8,9}. Also, there is now evidence to suggest that androstenediol may be of even greater importance as a promoter of breast tumour growth¹⁰. EMATE is also active *in vivo* as almost complete inhibition of rat liver E1-STS (99%) and DHA-STS (99%) activities resulted when it is administered either orally or subcutaneously¹¹. In addition, EMATE has been shown to have a memory enhancing effect in rats¹⁴. Studies in mice have suggested an association between DHA-STS activity and the regulation of part of the immune response. It is thought that this may also occur in humans^{15,16}. The bridging *O*-atom of the sulphamate moiety in EMATE is important for inhibitory activity. Thus, when the 3-*O*-atom is replaced by other heteroatoms (Figure 1) as in oestrone-3-*N*-sulphamate (4) and oestrone-3-S-sulphamate (5), these analogues are weaker non-time-dependent inactivators¹².

Although optimal potency for inhibition of E1-STS may have been attained in EMATE, it is possible that oestrone may be released during sulphatase inhibition^{8,12}, and that EMATE and its oestradiol congener may possess oestrogenic activity¹³.

The present invention seeks to provide novel compounds suitable for the inhibition of E1-STS but preferably wherein those compounds have no, or a minimal, oestrogenic effect.

According to a first aspect of the present invention there is provided a sulphamate compound suitable for use as an inhibitor of oestrone sulphatase, wherein the compound is defined as in claim 1.

The term "mimic" as used herein means having a similar or different structure but having a similar functional effect. In otherwords, group A and ring B together of the compounds of the present invention are bio-isosteres of the A and B rings of oestrone.

A key advantage of the present invention is that the sulphamate compounds of the present invention can act as E1-STS inhibitors.

Another advantage of the compounds of the present invention is that they may be potent *in vivo* and that they may have less oestrogenic activity than the known compounds and can therefore be deemed to be a "non-oestrogenic compound". The term "non-oestrogenic compound" as used herein means a compound exhibiting no or substantially no oestrogenic activity.

The present invention therefore provides sulphamate compounds which may have a reduced oestrogenic activity.

Another advantage is that the compounds may not be capable of being metabolised to compounds which display or induce hormonal activity.

The compounds of the present invention are also advantageous in that they may be orally active.

The compounds of the present invention are further advantageous in that they may have an irreversible effect.

In a preferred embodiment, the sulphamate compounds of the present invention are useful for the treatment of breast cancer.

In addition, the sulphamate compounds of the present invention are useful for the treatment of non-malignant conditions, such as the prevention of auto-immune diseases, particularly when pharmaceuticals may need to be administered from an early age.

The sulphamate compounds of the present invention are also believed to have therapeutic uses other than for the treatment of endocrine-dependent cancers, such as the treatment of autoimmune diseases.

Preferably, the compound has the Formula IV; wherein X is the sulphamate group; R₁ and/or R₂ is a substituent other than H; wherein R₁ and R₂ may be the same or different but not both being H; and wherein Y is CH₂.

The term "sulphamate" as used herein includes an ester of sulphamic acid, or an ester of an N-substituted derivative of sulphamic acid, or a salt thereof.

Preferably, the sulphamate group has the Formula III.

The term "hydrocarbyl group" as used herein means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. A non-limiting example of a hydrocarbyl group is an acyl group.

Here the term "hydrocarbon" means any one of an alkyl group, an alkenyl group, an alkynyl group, which groups may be linear, branched or cyclic, or an aryl group. The term hydrocarbon also includes those groups but wherein they have been optionally substituted. If the hydrocarbon is a branched structure having substituent(s) thereon, then the substitution may be on either the hydrocarbon backbone or on the branch; alternatively the substitutions may be on the hydrocarbon backbone and on the branch.

R₃ and R₄ are independently selected from H or alkyl, cycloalkyl, alkenyl and aryl, or together represent alkylene, wherein the or each alkyl or cycloalkyl or alkenyl or optionally contain one or more hetero atoms or groups.

When substituted, the N-substituted compounds of this invention may contain one or two N-alkyl, N-alkenyl, N-cycloalkyl or N-aryl substituents, preferably containing or each containing a maximum of 10 carbon atoms. When R₃ and/or R₄ is alkyl, the preferred values are those where R₃ and R₄ are each independently selected from lower alkyl groups containing from 1 to 5 carbon atoms, that is to say methyl, ethyl, propyl etc. Preferably R₃ and R₄ are both methyl. When R₃ and/or R₄ is aryl, typical values are phenyl and tolyl (-PhCH3; *o-*, *m-* or *p*-). Where R₃ and R₄ represent cycloalkyl, typical values are cyclopropyl, cyclopentyl, cyclohexyl etc. When joined together R₃ and R₄ typically represent an alkylene group providing a chain of 4 to 6 carbon atoms, optionally interrupted by one or more hetero atoms or groups, e.g. -0- or -NH- to provide a 5-, 6- or 7- membered heterocycle, e.g. morpholino, pyrrolidino or piperidino.

Within the values alkyl, cycloalkyl, alkenyl and aryl we include substituted groups containing as substituents therein one or more groups which do not interfere with the sulphatase inhibitory activity of the compound in question. Exemplary non-interfering substituents include hydroxy, amino, halo, alkoxy, alkyl and aryl.

In some preferred embodiments, at least one of R₃ and R₄ is H.

In some further preferred embodiments, each of R₃ and R₄ is H.

Preferably, each of R₁ and R₂ is independently selected from H, alkyl, cycloalkyl, alkoxy, alkenyl, aryl, substituted alkyl, substituted cycloalkyl, substituted alkenyl, substituted aryl, any other suitable hydrocarbyl group, a nitrogen containing group, a S containing group, a carboxy containing group.

Preferably, each of R₁ and R₂ is independently selected from H, C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms.

Likewise, here within the values alkyl, cycloalkyl, alkenyl and aryl we include substituted groups containing as substituents therein one or more groups which do not interfere with the sulphatase inhibitory activity of the compound in question. Exemplary non-interfering substituents include hydroxy, amino, halo, alkoxy, alkyl and aryl.

Preferably, each of R₁ and R₂ is independently selected from H, C₁₋₆ alkyl, C₁₋₆ alkenyl, a nitrogen containing group, or a carboxy group having from 1-6 carbon atoms.

Preferably, each of R₁ and R₂ is independently selected from H, C₁₋₆ alkyl, C₁₋₆ alkenyl, NO₂, or a carboxy group having from 1-6 carbon atoms.

Preferably, each of R₁ and R₂ is independently selected from H, C₃ alkyl, C₃ alkenyl, NO₂, or H₃CO.

Preferably, for some applications, the compound is further characterised by the feature that if the sulphamate group were to be substituted by a sulphate group to form a sulphate derivative, then the sulphate derivative would be hydrolysable by an enzyme having steroid sulphatase (E.C. 3.1.6.2) activity - i.e. when incubated with steroid sulphatase EC 3.1.6.2 at pH 7.4 and 37°C.

In one preferred embodiment, if the sulphamate group of the compound were to be replaced with a sulphate group to form a sulphate compound then that sulphate compound would be hydrolysable by an enzyme having steroid sulphatase (E.C. 3.1.6.2) activity and would yield a Km value of less than 50mmolar when incubated with steroid sulphatase EC 3.1.6.2 at pH 7.4 and 37°C.

In another preferred embodiment, if the sulphamate group of the compound were to be replaced with a sulphate group to form a sulphate compound then that sulphate compound would be hydrolysable by an enzyme having steroid sulphatase (E.C. 3.1.6.2) activity and would yield a Km value of less than 50µmolar when incubated with steroid sulphatase EC 3.1.6.2 at pH 7.4 and 37°C.

In a highly preferred embodiment, the compound of the present invention is not hydrolysable by an enzyme having steroid sulphatase (E.C. 3.1.6.2) activity.

Thus, the present invention provides novel sulphamate compounds.

When substituted, the N-substituted compounds of this invention may contain one or two N-alkyl, N-alkenyl, N-cycloalkyl or N-aryl substituents, preferably containing or each containing a maximum of 10 carbon atoms.

When R₁ and/or R₂ and/or R₃ and/or R₄ is alkyl, the preferred values are those where each of R₁ and R₂ and R₃ and R₄ is independently selected from lower alkyl groups containing from 1 to 6 carbon atoms, that is to say methyl, ethyl, propyl etc.

When R₁ and/or R₂ and/or R₃ and/or R₄ is aryl, typical groups are phenyl and tolyl (-PhCH₃; *o-*, *m-* or *p-*).

Where R₁ and/or R₂ and/or R₃ and/or R₄ represent cycloalkyl, typical values are cyclopropyl, cyclopentyl, cyclohexyl etc.

When joined together R₃ and R₄ typically represent an alkylene group providing a chain of 4 to 6 carbon atoms, optionally interrupted by one or more hetero atoms or groups, e.g. -0- or -NH- to provide a 5-, 6- or 7- membered heterocycle, e.g. morpholino, pyrrolidino or piperidino.

Within the values alkyl, cycloalkyl, alkenyl and aryl we include substituted groups containing as substituents therein one or more groups which do not interfere with the sulphatase inhibitory activity of the compound in question. Examples of non-interfering substituents include hydroxy, amino, halo, alkoxy, alkyl and aryl.

We have also surprisingly found that when the compound has the Formula IV where Y = - CH₂- it is not necessary for the compound to be substituted in the 2 and 4 ring positions, ie R₁ and R₂ may both be hydrogen. In one embodiment of this aspect, any of the ring positions (including R₁ and R₂, but excluding Y) may be substituted.

Thus, according to claim 1 there is provided a sulphamate compound suitable for use as an inhibitor of oestrone sulphatase wherein the compound has the Formula X and wherein X is a sulphamate group, and Y is CH₂ and optionally any other H attached directly to the ring system is substituted by another group.

X is as described in claim 1.

Any replacement for H on the ring system may be any one of the substituents described above in relation to R₁ and R₂.

In an especially preferred embodiment there is no substitution on the ring system, ie a compound of Formula IV where Y is -CH₂- and R₁ and R₂ are both H.

According to a second aspect of the present invention there is provided a sulphamate compound according to the present invention for use as a pharmaceutical.

According to a third aspect of the present invention there is provided a sulphamate compound according to the present invention for inhibiting oestrone sulphatase.

According to a fourth aspect of the present invention there is provided a pharmaceutical composition comprising a sulphamate compound according to the present invention; and a pharmaceutically acceptable carrier, excipient, adjuvant or diluent.

According to a fifth aspect of the present invention there is provided the use of a sulphamate compound according to the present invention in the manufacture of a pharmaceutical for inhibiting oestrone sulphatase.

The sulphamate compounds of the present invention may be prepared by reacting an appropriate alcohol with a sulfamoyl chloride, R₃R₄NSO₂Cl.

Preferred conditions for carrying out the reaction are as follows.

Sodium hydride and a sulfamoyl chloride are added to a stirred solution of the alcohol in anhydrous dimethyl formamide at 0°C. Subsequently, the reaction is allowed to warm to room temperature whereupon stirring is continued for a further 24 hours. The reaction mixture is poured onto a cold saturated solution of sodium bicarbonate and the resulting aqueous phase is extracted with dichloromethane. The combined organic extracts are dried over anhydrous MgSO₄. Filtration followed by solvent evaporation *in vacuo* and co-evaporated with toluene affords a crude residue which is further purified by flash chromatography.

Preferably, the alcohol is derivatised, as appropriate, prior to reaction with the sulfamoyl chloride. Where necessary, functional groups in the alcohol may be protected in known manner and the protecting group or groups removed at the end of the reaction.

For pharmaceutical administration, the steroid sulphatase inhibitors of this invention can be formulated in any suitable manner utilising conventional pharmaceutical formulating techniques and pharmaceutical carriers, adjuvants, excipients, diluents etc. and usually for parenteral administration. Approximate effective dose rates are in the range 100 to 800 mg/day depending on the individual activities of the compounds in question and for a patient of average (70Kg) bodyweight. More usual dosage rates for the preferred and more active compounds will be in the range 200 to 800 mg/day, more preferably, 200 to 500 mg/day, most preferably from 200 to 250 mg/day. They may be given in single dose regimes, split dose regimes and/or in multiple dose regimes lasting over several days. For oral administration they may be formulated in tablets, capsules, solution or suspension containing from 100 to 500 mg of compound per unit dose. Alternatively and preferably the compounds will be formulated for parenteral administration in a suitable parenterally administrable carrier and providing single daily dosage rates in the range 200 to 800 mg, preferably 200 to 500, more preferably 200 to 250 mg. Such effective daily doses will, however, vary depending on inherent activity of the active ingredient and on the bodyweight of the patient, such variations being within the skill and judgement of the physician.

For particular applications, it is envisaged that the steroid sulphatase inhibitors of this invention may be used in combination therapies, either with another sulphatase inhibitor, or, for example, in combination with an aromatase inhibitor, such as for example, 4-hydroxyandrostenedione (4-OHA).

In summation, the present invention provides novel compounds for use as steroid sulphatase inhibitors, and pharmaceutical compositions containing them.

The present invention will now be described only by way of example with reference to the accompanying drawings in which:-
Figure 1 shows the known structures of oestrone (1), oestrone sulphate (2), EMATE (3) and steroid sulphamates (4-5);
Figure 2 shows a compound of the Formula I;
Figure 3 shows a compound of the Formula II;
Figure 4 shows a compound of the Formula III;
Figure 5 shows a compound of the Formula IV;

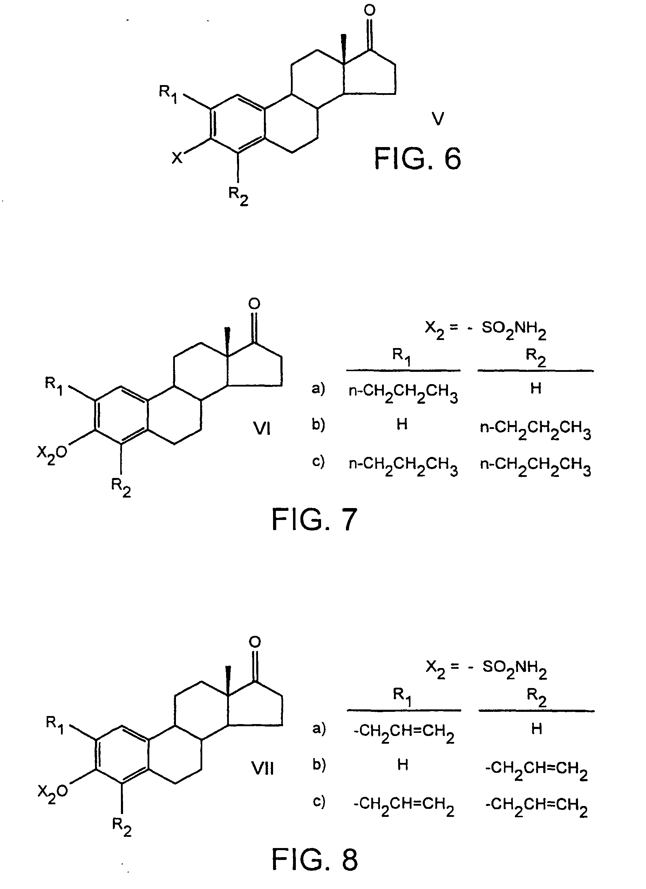

Figure 6 shows a compound of the Formula V;
Figure 7 shows a compound of the Formula VI;
Figure 8 shows a compound of the Formula VII;

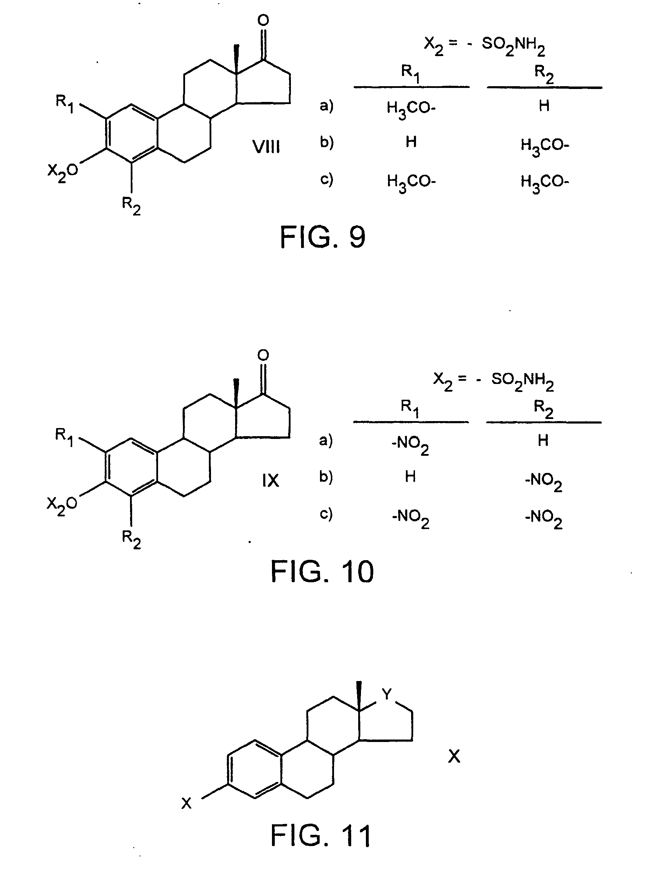

Figure 9 shows a compound of the Formula VIII;
Figure 10 shows a compound of the Formula IX;
Figure 11 shows a compound of the Formula X;
Figure 12 shows one embodiment of a method of preferring compounds of the present invention;
Figure 13 shows another embodiment of a method of preferring compounds of the present invention;
Figure 14 shows yet another embodiment of a method of preferring compounds of the present invention;
Figure 15 shows a further embodiment of a method of preferring compounds of the present invention;
Figure 16 shows a graph illustrating the *in vivo* inhibition of oestrone sulphatase by NOMATE (0.1 mg/Kg/day for five days); and
Figure 17 shows a graph illustrating the lack of effect of NOMATE (0.1 mg/Kg/day for five days) on uterine weights in ovariectomised rats.

The invention will now be described only by way of Examples.

### Example 1 - Preparative Methods

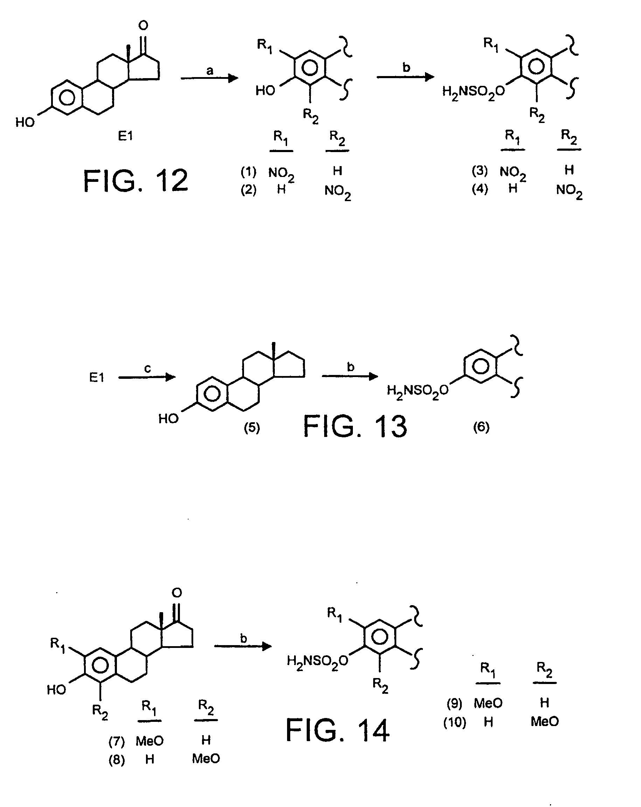

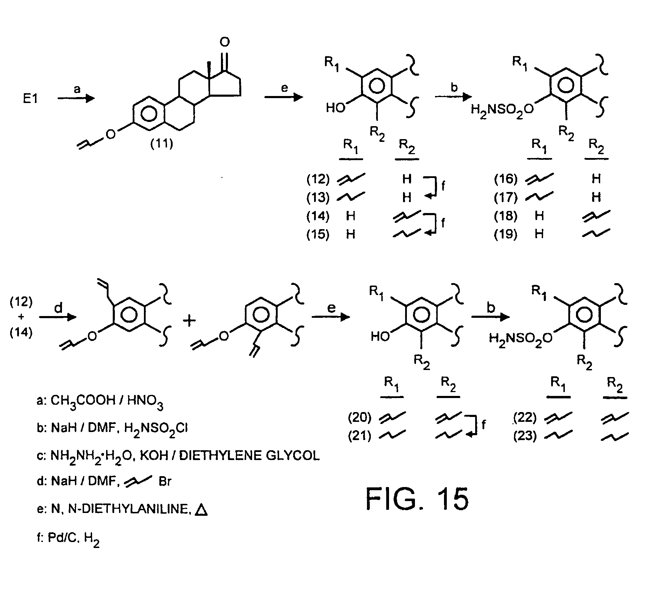

The preparation of various compounds in accordance with the present invention is illustrated in Figures 12 to 15. In these Figures, the curved lines attached to the phenyl rings represent the remainder of the ringed structure.

### Example 1 - In Vitro Inhibition

The ability of compounds to inhibit oestrone sulphatase activity was assessed using either intact MCF-7 breast cancer cells or placental microsomes as previously described¹¹.

In this regard, the teachings of that earlier reference¹¹ are as follows:

### Inhibition of Steroid Sulphatase Activity in MCF-7 cells by oestrone-3-sulphamate

Steroid sulphatase is defined as: Steryl Sulphatase EC 3.1.6.2.

Steroid sulphatase activity was measured *in vitro* using intact MCF-7 human breast cancer cells. This hormone dependent cell line is widely used to study the control of human breast cancer cell growth. It possesses significant steroid sulphatase activity (MacIndoe et al. Endocrinology, 123, 1281-1287 (1988); Purohit & Reed, Int. J. Cancer, 50, 901-905 (1992)) and is available in the U.S.A. from the American Type Culture Collection (ATCC) and in the U.K. (e.g. from The Imperial Cancer Research Fund). Cells were maintained in Minimal Essential Medium (MEM) (Flow Laboratories, Irvine, Scotland) containing 20 mM HEPES, 5% foetal bovine serum, 2 mM glutamine, non-essential amino acids and 0.075% sodium bicarbonate. Up to 30 replicate 25 cm² tissue culture flasks were seeded with approximately 1 x 10⁵ cells/flask using the above medium. Cells were grown to 80% confluency and medium was changed every third day.

Intact monolayers of MCF-7 cells in triplicate 25 cm² tissue culture flasks were washed with Earle's Balanced Salt Solution (EBSS from ICN Flow, High Wycombe, U.K.) and incubated for 3-4 hours at 37°C with 5 pmol (7 x 10⁵ dpm) [6,7-³H]oestrone-3-sulphate (specific activity 60 Ci/mmol from New England Nuclear, Boston, Mass., U.S.A.) in serum-free MEM (2.5 ml) together with oestrone-3-sulphamate (11 concentrations: 0; 1fM; 0.01pM; 0.1pM; 1PM; 0.01nM; 0.1nM; 1nM; 0.01mM; 0.1mM; 1mM). After incubation each flask was cooled and the medium (1 ml) was pipetted into separate tubes containing [¹⁴C]oestrone (7 x 10³ dpm) (specific activity 97 Ci/mmol from Amersham International Radiochemical Centre, Amersham, U.K.). The mixture was shaken thoroughly for 30 seconds with toluene (5 ml). Experiments showed that >90% [¹⁴C]oestrone and <0.1% [³H]oestrone-3-sulphate was removed from the aqueous phase by this treatment. A portion (2 ml) of the organic phase was removed, evaporated and the ³H and ¹⁴C content of the residue determined by scintillation spectrometry. The mass of oestrone-3-sulphate hydrolysed was calculated from the ³H counts obtained (corrected for the volumes of the medium and organic phase used, and for recovery of [¹⁴C]oestrone added) and the specific activity of the substrate. Each batch of experiments included incubations of microsomes prepared from a sulphatase-positive human placenta (positive control) and flasks without cells (to assess apparent non-enzymatic hydrolysis of the substrate). The number of cell nuclei per flask was determined using a Coulter Counter after treating the cell monolayers with Zaponin. One flask in each batch was used to assess cell membrane status and viability using the Trypan Blue exclusion method (Phillips, H.J. (1973) In: Tissue culture and applications, [eds: Kruse, D.F. & Patterson, M.K.]; pp. 406-408; Academic Press, New York).

Results for steroid sulphatase activity are expressed as the mean ± 1 S.D. of the total product (oestrone + oestradiol) formed during the incubation period (20 hours) calculated for 10⁶ cells and, for values showing statistical significance, as a percentage reduction (inhibition) over incubations containing no oestrone-3-sulphamate. Unpaired Student's t-test was used to test the statistical significance of results.

### Inhibition of Steroid Sulphatase Activity in Placental Microsomes by Oestrone-3-sulphamate

Sulphatase-positive human placenta from normal term pregnancies (Obstetric Ward, St. Mary's Hospital, London) were thoroughly minced with scissors and washed once with cold phosphate buffer (pH 7.4, 50 mM) then re-suspended in cold phosphate buffer (5 ml/g tissue). Homogenisation was accomplished with an Ultra-Turrax homogeniser, using three 10 second bursts separated by 2 minute cooling periods in ice. Nuclei and cell debris were removed by centrifuging (4°C) at 2000g for 30 minutes and portions (2 ml) of the supernatant were stored at -20°C. The protein concentration of the supernatants was determined by the method of Bradford (Anal. Biochem., 72, 248-254 (1976)).

Incubations (1 ml) were carried out using a protein concentration of 100 mg/ml, substrate concentration of 20 mM [6,7-³H]oestrone-3-sulphate (specific activity 60 Ci/mmol from New England Nuclear, Boston, Mass., U.S.A.) and an incubation time of 20 minutes at 37°C. If necessary eight concentrations of compounds are employed: 0 (i.e. control); 0.05mM; 0.1mM; 0.2mM; 0.4mM; 0.6mM; 0.8mM; 1.0mM. After incubation each sample was cooled and the medium (1 ml) was pipetted into separate tubes containing [¹⁴C]oestrone (7 x 10³ dpm) (specific activity 97 Ci/mmol from Amersham International Radiochemical Centre, Amersham, U.K.). The mixture was shaken thoroughly for 30 seconds with toluene (5 ml). Experiments showed that >90% [¹⁴C]oestrone and <0.1% [³H]oestrone-3-sulphate was removed from the aqueous phase by this treatment. A portion (2 ml) of the organic phase was removed, evaporated and the ³H and ¹⁴C content of the residue determined by scintillation spectrometry. The mass of oestrone-3-sulphate hydrolysed was calculated from the ³H counts obtained (corrected for the volumes of the medium and organic phase used, and for recovery of [¹⁴C]oestrone added) and the specific activity of the substrate.

For the present invention, the percentage inhibition for the series of EMATE analogues tested in either MCF-7 cells or placental microsomes is shown in Table 1.

### Example 2 - In Vivo Studies

Using 17-deoxy oestrone-3-O-sulphamate (NOMATE, Figure 5, Formula IV where X = - OSO₂NH₂, Y = -CH₂- and R₁ and R₂ = H, and Figure 13) as a representative example, the ability of this compound to inhibit oestrone sulphatase activity *in vivo* was examined in rats. The oestrogenicity of this compound was examined in ovariectomised rats. In this model compounds which are oestrogenic stimulate uterine growth.

### (i) Inhibition of oestrone sulphatase activity in vivo

NOMATE (0.1 mg/Kg/day for five days) was administered orally to rats with another group of animals receiving vehicle only (propylene glycol). At the end of the study samples of liver tissue were obtained and oestrone sulphatase activity assayed using ³H oestrone sulphate as the substrate as previously described¹¹.

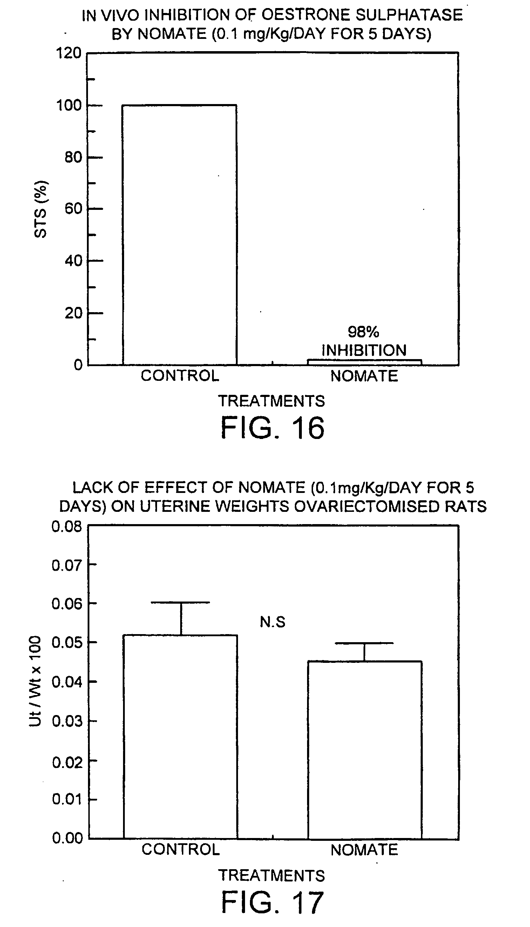

As shown in Figure 16, administration of this dose of NOMATE effectively inhibited oestrone sulphatase activity by 98% compared with untreated controls.

### (ii) Lack of in vivo oestrogenicity

NOMATE (0.1 mg/Kg/day for five days) was administered orally to rats with another group of animals receiving vehicle only (propylene glycol). At the end of the study uteri were obtained and weighed with the results being expressed as uterine weight/whole body weight x 100.

As shown in Figure 17, administration of NOMATE at the dose tested, but had no significant effect on uterine growth, showing that at this dose the compound is not oestrogenic.

**TABLE 1**

| **Inhibition of Oestrone Sulphatase Activity in MCF-7 Cells or Placental Microsomes by EMATE Analogues** | | | |
|---|---|---|---|
| **Inhibitor** | **Concentration** | **% Inhibition (Mean)** | |
| | **Tested (mM)** | **MCF-7 Cells** | **Placental Microsomes** |
| 2-n-propyl EMATE | 0.1 | 41.1 | - |
| | 1 | 83.1 | 21.9 |
| | 10 | 92.2 | 43.2 |
| | 25 | - | 47.5 |
| | 50 | - | 61.1 |
| | 100 | - | 69.2 |
| 4-n-propyl EMATE | 1 | - | 13.7 |
| | 10 | - | 10.2 |
| | 25 | - | 15.7 |
| | 50 | - | 16.3 |
| | 100 | - | 23.7 |
| 2,4-n-dipropyl EMATE | 0.1 | 6.6 | - |
| | 1 | 10.6 | - |
| 2-allyl EMATE | 0.01 | 23.2 | - |
| | 0.1 | 76.1 | - |
| | 1 | 94.2 | 45.6 |
| | 10 | 93.7 | 65.4 |
| | 25 | - | 75.3 |
| | 50 | - | 86.6 |
| | 100 | - | 89.6 |
| 4-allyl EMATE (approx 75%) | 1 | - | 29.1 |
| | 10 | - | 54.2 |
| | 25 | - | 59.0 |
| | 50 | - | 65.1 |
| | 100 | - | 71.9 |
| 2,4-di-allyl EMATE | - | - | - |
| 2-methoxy EMATE | 0.1 | 96.0 | - |
| | 1 | 93.6 | - |
| | 10 | 96.2 | 99.0 |
| | 50 | - | 99.7 |
| | 100 | - | 99.7 |
| 2-nitro EMATE | 0.05 | - | 44.5 |
| | 0.5 | - | 93.9 |
| | 5 | - | 99.0 |
| | 50 | - | 99.4 |
| 4-nitro EMATE NOMATE (17-deoxy EMATE) | 20 | - | 99.0 |
| | 0.1 | 96.4 | 97.2 |
| | 1 | 99.1 | 99.5 |
| | 10 | 99.7 | 99.5 |
| | 25 | 99.7 | 99.7 |

| | | | |
|---|---|---|---|
| -- = not tested - Irreversible time- and concentration-dependent inhibition is assumed for these compounds in keeping with established precedent⁸. | | | |

Other modifications of the present invention will be apparent to those skilled in the art.

### REFERENCES

(1) Santner, S. J.; Feil, P. D.; Santen, R. J. In situ oestrogen production via the oestrone sulphatase pathway in breast tumors: relative importance vs. the aromatase pathway. J. Clin. Endocrinol. Metab. 1984, 59, 29-33.
(2) Yamamoto, T.; Kitawaki, J.; Urabe, M.; Honjo, H.; Tamura, T.; Noguchi, T.; Okada, H.; Sasaki, H.; Tada, A.; Terashima, Y.; Nakamura, J.; Yoshihama, M. Oestrogen productivity of endometrium and endometrial cancer tissue - influence of aromatase on proliferation of endometrial cancer cells. J. Steroid Biochem. Mol. Biol. 1993, 44, 463-468.
(3) Santen, R. J.; Santner, S. J.; Davis, B.; Veldhuis, J.; Samojilik, E.; Ruby, E. Aminogluthethimide inhibits extraglandular oestrogen production in post-menopausal women with breast carcinoma. J. Clin. Endocrinol. Metab. 1978, 47, 1257-1265.
(4) Reed, M. J.; Lai, L. C.; Owen, A. M.; Singh, A.; Coldham, N. G.; Purohit, A.; Ghilchik, M. W.; Shaikh, N. A.; James, V. H. T. Effect of treatment with 4-hydroxyandrostenedione on the peripheral conversion of androstenedione to oestrone and in vitro tumour aromatase activity in postmenopausal women with breast cancer. Cancer Res. 1990, 50, 193-196.
(5) Ruder, H. J.; Loriaux, D. L.; Lipsett, M. B. Oestrone sulphate: production rate and metabolism in man. J. Clin. Invest. 1972, 51, 1020-1023.
(6) James, V. H. T.; McNeill, J. M.; Lai, L. C.; Newton, C. J.; Ghilchik, M. W.; Reed, M. J. Aromatase activity in normal breast and breast tumor tissues: in vivo and in vitro studies. Steroids 1987, 50, 269-279.
(7) Howarth, N. M.; Purohit, A.; Reed, M. J.; Potter, B. V. L. Oestrone sulphamates: potent inhibitors of oestrone sulphatase with therapeutic potential. J. Med. Chem. 1994, 37, 219-221.
(8) Purohit, A.; Williams, G. J.; Howarth, N. M.; Potter, B. V. L.; Reed, M. J. Inactivation of steroid sulphatase by an active site-directed inhibitor, oestrone-3-O-sulphamate. Biochemistry 1995, 34, 11508-11514.
(9) Purohit, A.; Dauvois, S.; Parker, M. G.; Potter, B. V. L.; Williams, G. J.; Reed, M. J. The hydrolysis of oestrone sulphate and dehydroepiandrosterone sulphate by human steroid sulphatase expressed in transfected COS-1 cells. J. Steroid Biochem. Mol. Biol. 1994, 50, 101-104.
(10) Dauvois, S.; Labrie, F. Androstenedione and androst-5-ene-3b,17b-diol stimulate DMBA-induced rat mammary tumours - role of aromatase. Breast Cancer Res. Treat. 1989, 13, 61-69.
(11) Purohit, A.; Williams, G. J.; Roberts, C. J.; Potter, B. V. L.; Reed, M. J. In vivo inhibition of oestrone sulphatase and dehydroepiandrosterone sulphatase by oestrone-3-O-sulphamate. Int. J. Cancer 1995, 62, 106-111.
(12) Woo, L. W. L.; Lightowler, M.; Purohit, A.; Reed, M. J.; Potter, B. V. L. Heteroatom-substituted analogues of the active-site directed inhibitor oestra-1,3,5(10)-trien-17-one-3-sulphamate inhibit oestrone sulphatase by a different mechanism. J. Steroid Biochem. Mol. Biol. 1996 (in press).
(13) Elger, W.; Schwarz, S.; Hedden, A.; Reddersen, G.; Schneider, B. Sulphamates of various oestrogens - prodrugs with increased systemic and reduced hepatic oestrogenicity at oral application. J. Steroid Biochem. Mol. Biol. 1995, 55, 395-403.
(14) Li, P. K; Rhodes, M. E.; Jagannathan, S; Johnson, D. A. Memory enhancement mediated by the steroid sulphatase inhibitor oestrone 3-O-sulphamate. J. Endocrinol. 1995, 144, Abstr. P155.
(15) Daynes, R. A.; Araneo, B. A.; Dowell, T. A.; Huang, K.; Dudley, D. Regulation of murine lymphokine production in vivo. 3. The lymphoid tissue micro-environment exerts regulatory influences over T-helper cell function. J Exp. Med. 1990, 171, 979-996.
(16) Rook, G. A. W.; Hernandez-Pando, R.; Lightman, S. Hormones, peripherally activated prohormones and regulation of the TH1/TH2 balance. Immunol. Today 1994, 15, 301-303.

## Claims

1. A sulphamate compound suitable for use as an inhibitor of oestrone sulphatase wherein the compound has Formula X 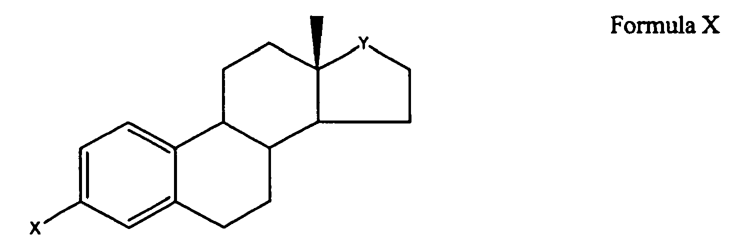 and wherein X is a sulphamate group, and Y is CH₂ and optionally any other H attached directly to the ring system is substituted by another group.

2. A sulphamate compound according to claim 1 wherein at least one of the other H atoms attached directly to the ring system is subsituted and each substituent is independently selected from alkyl, cycloalkyl, alkenyl, aryl, substituted alkyl, substituted cycloalkyl, substituted alkenyl, substituted aryl, a nitrogen containing group, an S containing group or a carboxy containing group.

3. A sulphamate compound according to claim 2 wherein each substituent is independently selected from C₁₋₆ alkyl, C₁₋₆ cycloalkyl, C₁₋₆ alkenyl, substituted C₁₋₆ alkyl, substituted cycloalkyl, substituted C₁₋₆ alkenyl, substituted aryl, NO₂, or a carboxy group having from 1-6 carbon atoms.

4. A sulphamate compound according to claim 3 wherein each substituent is independently selected from C₃ alkyl or C₃ alkenyl.

5. A sulphamate compound according to any one of claims 1 to 4 wherein the sulphamate group has the Formula III; 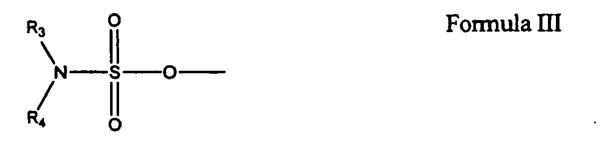 wherein each of R₃ and R₄ is independently selected from H, alkyl, cycloalkyl, alkenyl and aryl, or together represent alkylene optionally containing one or more hetero atoms or groups in the alkylene chain.

6. A sulphamate compound according to claim 5 wherein at least one of R₃ and R₄ is H.

7. A sulphamate compound according to claim 6 wherein each of R₃ and R₄ is H.

8. A sulphamate compound according to claim 1 wherein none of the H atoms attached directly to the ring system is substituted.

9. A sulphamate compound according to any one of claims 1 to 7 wherein the compound has the Formula IV; 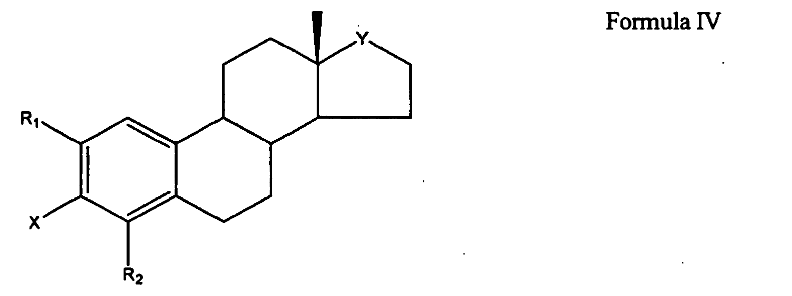 wherein each of R₁ and R₂ is independently selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group;
R₁ and R₂ may be the same or different but not both being H.

10. A sulphamate compound according to claim 9 wherein each of R₁ and R₂ is independently selected from H, C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms.

11. A sulphamate compound according to claim 10 wherein each of R₁ and R₂ is independently selected from H, C₁₋₆ alkyl, C₁₋₆ alkenyl, a nitrogen containing group, or a carboxy group having from 1-6 carbon atoms.

12. A sulphamate compound according to claim 11 wherein each of R₁ and R₂ is independently selected from H, C₁₋₆ alkyl, C₁₋₆ alkenyl, NO₂, or a carboxy group having from 1-6 carbon atoms.

13. A sulphamate compound according to claim 12 wherein each of R₁ and R₂ is independently selected from H, C₃ alkyl, C₃ alkenyl, or NO₂.

14. A sulphamate compound according to claim 9 wherein R₁ and/or R₂ is an alkoxy group.

15. A sulphamate compound according to claim 14 wherein R₁ and/or R₂ is a methoxy group.

16. A sulphamate compound according to claim 14 wherein R₁ is an alkoxy group.

17. A sulphamate compound according to claim 16 wherein R₁ is a methoxy group.

18. A sulphamate compound according to claim 9 wherein R₁ and/or R₂ is an alkyl group.

19. A sulphamate compound according to claim 18 wherein R₁ and/or R₂ is a C₁₋₆ alkyl group.

20. A sulphamate compound according to claim 19 wherein R₁ and/or R₂ is an ethyl group.

21. A sulphamate compound according to any one of the preceding claims for use as a pharmaceutical.

22. A sulphamate compound according to any one of claims 1 to 20 for inhibiting oestrone sulphatase.

23. A pharmaceutical composition comprising a sulphamate compound according to any one of claims 1 to 20; and a pharmaceutically acceptable carrier, adjuvant, excipient or diluent.

24. Use of a sulphamate compound according to any one of claims 1 to 20 in the manufacture of a pharmaceutical for inhibiting oestrone sulphatase.

## Patentansprüche

1. Sulfamatverbindung, die zur Verwendung als Östronsulfataseinhibitor geeignet ist, wobei die Verbindung die Formel X hat 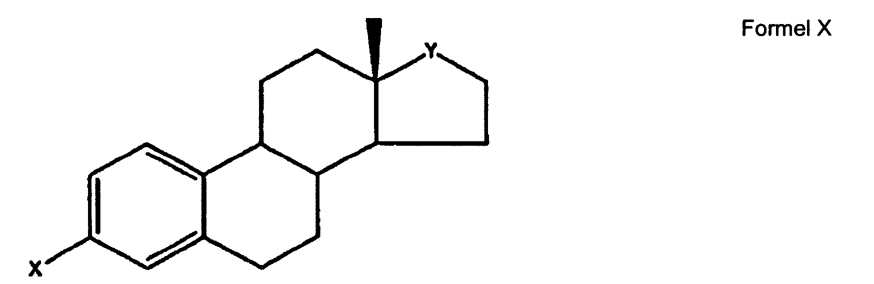 und wobei X eine Sulfamatgruppe ist und Y CH₂ ist und optional irgendein weiterer H, der direkt an das Ringsystem angebunden ist, durch eine weitere Gruppe substituiert ist.

2. Sulfamatverbindung nach Anspruch 1, wobei wenigstens eines der weiteren H-Atome, die direkt an das Ringsystem angebunden sind, substituiert ist und jeder Substituent unabhängig voneinander ausgewählt ist unter Alkyl, Cycloalkyl, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe.

3. Sulfamatverbindung nach Anspruch 2, wobei jeder Substituent unabhängig voneinander ausgewählt ist unter C₁₋₆-Alkyl, C₁₋₆-Cycloalkyl, C₁₋₆-Alkenyl, substituiertem C₁₋₆-Alkyl, substituiertem Cycloalkyl, substituiertem C₁₋₆-Alkenyl, substituiertem Aryl, NO₂ oder einer Carboxygruppe mit 1-6 Kohlenstoffatomen.

4. Sulfamatverbindung nach Anspruch 3, wobei jeder Substituent unabhängig voneinander unter C₃-Alkyl oder C₃-Alkenyl ausgewählt ist.

5. Sulfamatverbindung nach einem der Ansprüche 1 bis 4, wobei die Sulfamatgruppe die Formel III hat 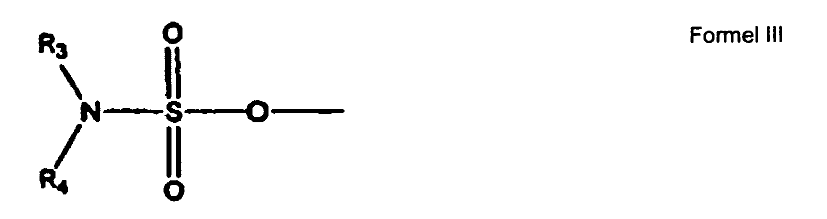 wobei jeder von R₃ und R₄ unabhängig voneinander ausgewählt ist unter H, Alkyl, Cycloalkyl, Alkenyl und Aryl oder sie zusammen Alkylen, welches optional ein oder mehrere Heteroatome oder -gruppen in der Alkylenkette enthält, repräsentieren.

6. Sulfamatverbindung nach Anspruch 5, wobei wenigstens einer von R₃ und R₄ H ist.

7. Sulfamatverbindung nach Anspruch 6, wobei jeder von R₃ und R₄ H ist.

8. Sulfamatverbindung nach Anspruch 1, wobei keines der direkt an das Ringsystem angebundenen H-Atome substituiert ist.

9. Sulfamatverbindung nach einem der Ansprüche 1 bis 7, wobei die Verbindung die Formel IV hat 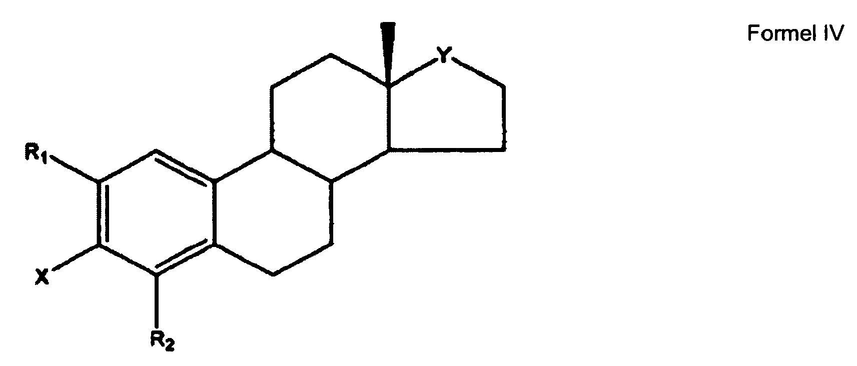 wobei jeder von R₁ und R₂ unabhängig voneinander ausgewählt ist unter H, Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe,
R₁ und R₂ gleich oder verschieden sein können, jedoch nicht beide H sind.

10. Sulfamatverbindung nach Anspruch 9, wobei jeder von R₁ und R₂ unabhängig voneinander ausgewählt ist unter H, C₁₋₆-Alkyl, C₁₋₆-Cycloalkyl, C₁₋₆-Alkenyl, substituiertem C₁₋₆-Alkyl, substituiertem C₁₋₆-Cycloalkyl, substituiertem C₁₋₆-Alkenyl, substituiertem Aryl, einer Stickstoff enthaltenden Gruppe, einer S enthaltenden Gruppe oder einer Carboxygruppe mit 1-6 Kohlenstoffatomen.

11. Sulfamatverbindung nach Anspruch 10, wobei jeder von R₁ und R₂ unabhängig voneinander ausgewählt ist unter H, C₁₋₆-Alkyl, C₁₋₆-Alkenyl, einer Stickstoff enthaltenden Gruppe oder einer Carboxygruppe mit 1-6 Kohlenstoffatomen.

12. Sulfamatverbindung nach Anspruch 11, wobei jeder von R₁ und R₂ unabhängig voneinander ausgewählt ist unter H, C₁₋₆-Alkyl, C₁₋₆-Alkenyl, NO₂ oder einer Carboxygruppe mit 1-6 Kohlenstoffatomen.

13. Sulfamatverbindung nach Anspruch 12, wobei jeder von R₁ und R₂ unabhängig voneinander ausgewählt ist unter H, C₃-Alkyl, C₃-Alkenyl oder NO₂.

14. Sulfamatverbindung nach Anspruch 9, wobei R₁ und/oder R₂ eine Alkoxygruppe ist.

15. Sulfamatverbindung nach Anspruch 14, wobei R₁ und/oder R₂ eine Methoxygruppe ist.

16. Sulfamatverbindung nach Anspruch 14, wobei R₁ eine Alkoxygruppe ist.

17. Sulfamatverbindung nach Anspruch 16, wobei R₁ eine Methoxygruppe ist.

18. Sulfamatverbindung nach Anspruch 9, wobei R₁ und/oder R₂ eine Alkylgruppe ist.

19. Sulfamatverbindung nach Anspruch 18, wobei R₁ und/oder R₂ eine C₁₋₆-Alkylgruppe ist.

20. Sulfamatverbindung nach Anspruch 19, wobei R₁ und/oder R₂ eine Ethylgruppe ist.

21. Sulfamatverbindung nach einem der vorangegangenen Ansprüche zur Verwendung als Arzneimittel.

22. Sulfamatverbindung nach einem der Ansprüche 1 bis 20 zur Hemmung von Östronsulfatase.

23. Pharmazeutische Zusammensetzung, welche eine Sulfamatverbindung nach einem der Ansprüche 1 bis 20 und einen pharmazeutisch verträglichen Träger, ein Adjuvans, einen Hilfsstoff oder ein Verdünnungsmittel umfaßt.

24. Verwendung einer Sulfamatverbindung nach einem der Ansprüche 1 bis 20 bei der Herstellung eines Arzneimittels zum Hemmen von Östronsulfatase.

## Revendications

1. Composé sulfamate apte à l'utilisation comme inhibiteur d'oestrone-sulfatase, le composé répondant à la formule X 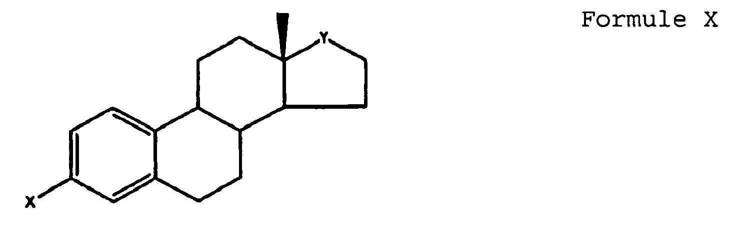 dans laquelle X représente un groupe sulfamate et Y représente un groupe CH₂ et, facultativement, n'importe quel autre atome de H fixé directement au système cyclique est substitué par un autre groupe.

2. Composé sulfamate suivant la revendication 1, dans lequel au moins un des autres atomes de H fixés directement au système cyclique est substitué et chaque substituant est choisi indépendamment entre des groupes alkyle, cycloalkyle, alcényle, aryle, alkyle substitué, cycloalkyle substitué, alcényle substitué, aryle substitué, un groupe azoté et un groupe contenant S et un groupe à fonction carboxy.

3. Composé sulfamate suivant la revendication 2, dans lequel chaque substituant est choisi indépendamment entre des groupes alkyle en C₁ à C₆, cycloalkyle en C₁ à C₆, alcényle en C₁ à C₆, alkyle en C₁ à C₆ substitué, cycloalkyle substitué, alcényle en C₁ à C₆ substitué, aryle substitué, NO₂, et un groupe carboxy ayant 1 à 6 atomes de carbone.

4. Composé sulfamate suivant la revendication 3, dans lequel chaque substituant est choisi indépendamment entre des groupes alkyle, en C₃ et alcényle en C₃.

5. Composé sulfamate suivant l'une quelconque des revendications 1 à 4, dans lequel le groupe sulfamate répond à la formule III : 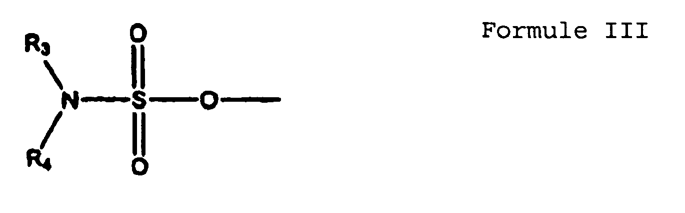 dans laquelle chacun de R₃ et R₄ est choisi indépendamment entre H, des groupes alkyle, cycloalkyle, alcényle et aryle, ou bien ils représentent conjointement un groupe alkylène contenant facultativement un ou plusieurs hétéroatomes ou hétérogroupes dans la chaîne alkylène.

6. Composé sulfamate suivant la revendication 5, dans laquelle l'un de R₃ et R₄ représente un atome de H.

7. Composé sulfamate suivant la revendication 6, dans lequel chacun de R₃ et R₄ représente un atome de H.

8. Composé sulfamate suivant la revendication 1, dans lequel aucun des atomes de H fixés directement au système cyclique n'est substitué.

9. Composé sulfamate suivant l'une quelconque des revendications 1 à 7, le composé répondant à la formule IV: 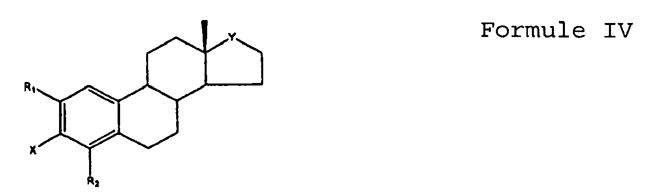 dans laquelle chacun de R₁ et R₂ est choisi indépendamment entre H, des groupes alkyle, cycloalkyle, alkoxy, alcényle, aryle, alkyle substitué, cycloalkyle substitué, alcényle substitué, aryle substitué, un groupe azoté, un groupe contenant S et un groupe à fonction carboxy ;
R₁ et R₂ peuvent être identiques ou différents mais ne représentent ni l'un ni l'autre un atome de H.

10. Composé sulfamate suivant la revendication 9, dans lequel chacun de R₁ et R₂ est choisi indépendamment entre H, des groupes alkyle en C₁ à C₆, cycloalkyle en C₁ à C₆, alcényle en C₁ à C₆, alkyle en C₁ à C₆ substitué, cycloalkyle en C₁ à C₆ substitué, alcényle en C₁ à C₆ substitué, aryle substitué, un groupe azoté, un groupe contenant S et un groupe carboxy ayant 1 à 6 atomes de carbone.

11. Composé sulfamate suivant la revendication 10, dans lequel chacun de R₁ et R₂ est choisi indépendamment entre H, des groupes alkyle en C₁ à C₆, alcényle en C₁ à C₆, un groupe azoté et un groupe carboxy ayant 1 à 6 atomes de carbone.

12. Composé sulfamate suivant la revendication 11, dans lequel chacun de R₁ et R₂ est choisi indépendamment entre H, des groupes alkyle en C₁ à C₆, alcényle en C₁ à C₆, NO₂ et un groupe carboxy ayant 1 à 6 atomes de carbone.

13. Composé sulfamate suivant la revendication 12, dans lequel chacun de R₁ et R₂ est choisi indépendamment entre H, des groupes alkyle en C₃, alcényle en C₃ et NO₂.

14. Composé sulfamate suivant la revendication 9, dans lequel R₁ et/ou R₂ représente un groupe alkoxy.

15. Composé sulfamate suivant la revendication 14, dans lequel R₁ et/ou R₂ représente un groupe méthoxy.

16. Composé sulfamate suivant la revendication 14, dans lequel R₁ représente un groupe alkoxy.

17. Composé sulfamate suivant la revendication 16, dans lequel R₁ représente un groupe méthoxy.

18. Composé sulfamate suivant la revendication 9, dans lequel R₁ et/ou R₂ représente un groupe alkyle.

19. Composé sulfamate suivant la revendication 18, dans lequel R₁ et/ou R₂ représente un groupe alkyle en C₁ à C₆.

20. Composé sulfamate suivant la revendication 19, dans lequel R₁ et/ou R₂ représentent un groupe éthyle.

21. Composé sulfamate suivant l'une quelconque des revendications précédentes, destiné à être utilisé comme agent pharmaceutique.

22. Composé sulfamate suivant l'une quelconque des revendications 1 à 20, destiné à inhiber l'oestrone-sulfatase.

23. Composition pharmaceutique comprenant un composé sulfamate suivant l'une quelconque des revendications 1 à 20, et un support, adjuvant, excipient ou diluant pharmaceutiquement acceptable.

24. Utilisation d'un composé sulfamate suivant l'une quelconque des revendications 1 à 20, dans la production d'un agent pharmaceutique destiné à inhiber l'oestrone-sulfatase.
